# EUROPEAN PATENT SPECIFICATION

(11) **EP 1 931 762 B1**
(45) Date of publication and mention of the grant of the patent: **05.12.2012**
(21) Application number: 06806911.1
(22) Date of filing: 02.10.2006
(51) Int. Cl.: C12N 1/16, C12N 1/18, A61K 48/00, A61P 29/00

(54) **USE OF RECOMBINANT YEAST STRAIN PRODUCING AN ANTI -INFLAMMATORY COMPOUND IN THE MANUFACTURE OF A MEDICAMENT TO TREAT COLITIS**
VERWENDUNG EINES EINE ENTZÜNDUNGSHEMMENDE VERBINDUNG PRODUZIERENDEN REKOMBINANTEN HEFESTAMMS BEI DER HERSTELLUNG EINES ARZNEIMITTELS ZUR BEHANDLUNG VON COLITIS
UTILISATION D'UNE SOUCHE DE LEVURE RECOMBINANTE PRODUISANT UN COMPOSE ANTI-INFLAMMATOIRE POUR LA FABRICATION D'UN MEDICAMENT DESTINE AU TRAITEMENT DE LA COLITE

(30) Priority: 03.10.2005 EP 05109153
(43) Date of publication of application: 18.06.2008
(73) Proprietor: Actogenix N.V., 9052 Zwijnaarde (BE)
(72) Inventor: ROTTIERS, Pieter, B-9840 De Pinte (BE); VANDENBROUCKE, Klaas, B-9000 Gent (BE); ISERENTANT, Dirk, B-3018 Wijgmaal (BE)
(74) Representative: Michalík, Andrej
(86) International application number: PCT/EP2006/066950
(87) International publication number: WO 2007/039586

(56) References cited:
- WO-A1-01/98461
- WO-A2-00/23471
- WO-A2-03/064607
- BLANQUET STEPHANIE ET AL: "Living recombinant Saccharomyces cerevisiae secreting proteins or peptides as a new drug delivery system in the gut" JOURNAL OF BIOTECHNOLOGY, vol. 110, no. 1, 13 May 2004 (2004-05-13), pages 37-49, XP002412583 ISSN: 0168-1656 cited in the application
- DATABASE MEDLINE [Online] US NATIONAL LIBRARY OF MEDICINE (NLM), BETHESDA, MD, US; January 2005 (2005-01), SANTELMANN HEIKO ET AL: "Yeast metabolic products, yeast antigens and yeasts as possible triggers for irritable bowel syndrome." XP002412586 Database accession no. NLM15647635 & EUROPEAN JOURNAL OF GASTROENTEROLOGY & HEPATOLOGY. JAN 2005, vol. 17, no. 1, January 2005 (2005-01), pages 21-26, ISSN: 0954-691X
- CROXFORD J L ET AL: "Different therapeutic outcomes in experimental allergic encephalomyelitis dependant upon the mode of delivery of IL-10: A comparison of the effects of protein, adenoviral or retroviral IL-10 delivery into the central nervous system" JOURNAL OF IMMUNOLOGY 15 MAR 2001 UNITED STATES, vol. 166, no. 6, 15 March 2001 (2001-03-15), pages 4124-4130, XP002412584 ISSN: 0022-1767
- DATABASE BIOSIS [Online] BIOSCIENCES INFORMATION SERVICE, PHILADELPHIA, PA, US April 2005 DALMASSO GUILLAUME ET AL: 'Saccharomyces boulardii prevents and ameliorates trinitrobenzene sulfonic acid-induced colitis in mice.' Database accession no. PREV200600212076 & GASTROENTEROLOGY, vol. 128, no. 4, Suppl. 2, April 2005 (2005-04), page A618, DIGESTIVE DISEASE WEEK MEETING/106TH ANNUAL MEETING OF THE AMERICAN-GASTROENTEROLOGICAL-ASSOCIATION; CHICAGO, IL, USA; MAY 14 19, 2005 ISSN: 0016-5085
- DATABASE MEDLINE [Online] US NATIONAL LIBRARY OF MEDICINE (NLM), BETHESDA, MD, US June 2003 GUSLANDI MARIO ET AL: 'A pilot trial of Saccharomyces boulardii in ulcerative colitis.' Database accession no. NLM12840682 & GUSLANDI MARIO ET AL: "A pilot trial of Saccharomyces boulardii in ulcerative colitis.", EUROPEAN JOURNAL OF GASTROENTEROLOGY & HEPATOLOGY JUN 2003 LNKD- PUBMED:12840682, vol. 15, no. 6, June 2003 (2003-06), pages 697-698, ISSN: 0954-691X
- J PATHOL, vol. 199, 2003, pages 361-367,
- GUSLANDI MARIO ET AL: "Saccharomyces boulardii in maintenance treatment of Crohn's disease", DIGESTIVE DISEASES AND SCIENCES, PLENUM PUBLISHING CO, US, vol. 45, no. 7, 1 July 2000 (2000-07-01), pages 1462-1464, XP002503019, ISSN: 0163-2116, DOI: 10.1023/A:1005588911207

### SEQUENCE LISTING

<110> VIB vzw
   Universiteit Gent
<120> USE CF A RECOMBINANT YEAST STRAIN PRODUCING AN ANTI-INFLAMMATORY COMPOUND TO TREAT COLITIS
<130> PRO/ydl/V219
<150> EP05109153.6
   <151> 2005- 10- 03
<160> 15
<170> Patent In version 3. 3
<210> 1
   <211> 21
   <212> DNA
   <213> Artificial
<220>
   <223> Oligo ml L 10 S
<400> 1
   cagt acagcc gggaagacaa t 21
<210> 2
   <211> 25
   <212> DNA
   <213> Artificial
<220>
   <223> Oligo ml L10 AS
<400> 2
   gcact agtt a gcttttcatttt gat 25
<210> 3
   <211> 38
   <212> DNA
   <213> Artificial
<220>
   <223> Oligo Spel - TPI - S
<400> 3
   gcact agt at ccgagattat atct aggaac ccat cagg 38
<210> 4
   <211> 31
   <212> DNA
   <213> Artificial
<220>
   <223> Oligo ppMF-middle- AS
<400> 4
   ct t ct aaat ctgagtaaccg atgacagctt c 31
<210> 5
   <211> 28
   <212> DNA
   <213> Artificial
<220>
   <223> Oligo ppMF-start-S
<400> 5
   atgagatttc cttcaatttt tactgcag 28
<210> 6
   <211> 30
   <212> DNA
   <213> Artificial
<220>
   <223> Oligo ml L10- EcoRl - middle- AS
<400> 6
   cagggaat t caaatgctccttgatttctgg 30
<210> 7
   <211> 157
   <212> PRT
   <213> Mus musculus
<400> 7
   Gln Tyr Ser Arg Glu Asp Asn Asn Cys Thr His Phe Pro Val Gly Gln
   1               5                   10                  15
   Ser His Met Leu Leu Glu Leu Arg Thr Ala Phe Ser Gln Val Lys Thr
               20                  25                  30
   Phe Phe Gln Thr Lys Asp Gln Leu Asp Asn Ile Leu Leu Thr Asp Ser
           35                  40                  45
   Leu Met Gln Asp Phe Lys Gly Tyr Leu Gly Cys Gln Ala Leu Ser Glu
       50                  55                  60
   Met Ile Gln Phe Tyr Leu Val Glu Val Met Pro Gln Ala Glu Lys His
   65                  70                  75                  80
   Gly Pro Glu Ile Lys Glu His Leu Asn Ser Leu Gly Glu Lys Leu Lys
                   85                  90                  95
   Thr Leu Arg Met Arg Leu Arg Arg Cys His Arg Phe Leu Pro Cys Glu
               100                 105                 110
   Asn Lys Ser Lys Ala Val Glu Gln Val Lys Ser Asp Phe Asn Lys Leu
           115                 120                 125
   Gln Asp Gln Gly Val Tyr Lys Ala Met Asn Glu Phe Asp Ile Phe Ile
       130                 135                 140
   Asn Cys Ile Glu Ala Tyr Met Met Ile Lys Met Lys Ser
   145                 150                 155
<210> 8
   <211> 72
   <212> DNA
   <213> Artificial
<220>
   <223> Oligo 5' URA3P-TPI-S
<400> 8
   ttttgaccat caaagaaggt taatgtggct gtggtttcag ggtccataga tttccttcaa 60
   tttttactgc ag 72
<210> 9
   <211> 77
   <212> DNA
   <213> Artificial
<220>
   <223> ml L10-3' URA3-AS
<400> 9
   ct aat t t gt g agt t t agt at acat gcat t t act t at aat a cagt t t t t t a gct t t t cat t 60
   t t gat cat ca t gt at gc 77
<210> 10
   <211> 20
   <212> DNA
   <213> Artificial
<220>
   <223> URA3-S
<400> 10
   tgctgctact catcctagtc 20
<210> 11
   <211> 20
   <212> DNA
   <213> Artificial
<220>
   <223> URA3-AS
<400> 11
   tcatctcttc cacccat gt c 20
<210> 12
   <211> 20
   <212> DNA
   <213> Artificial
<220>
   <223> 5' URA3 flanking-S
<400> 12
   attgagggcg gat t act acc 20
<210> 13
   <211> 20
   <212> DNA
   <213> Artificial
<220>
   <223> ml L10AS
<400> 13
   aggagt cggt t agcagt at g 20
<210> 14
   <211> 20
   <212> DNA
   <213> Artificial
<220>
   <223> ml L10-S
<400> 14
   gcagtggagc aggtgaagag 20
<210> 15
   <211> 20
   <212> DNA
   <213> Artificial
<220>
   <223> 3' URA3 flanking-AS
<400> 15
   cggttgttcc gtttgacttg 20

## Description

The invention generally relates to compositions for the delivery at the intestinal mucosa of anti-inflammatory compounds, preferably of acid sensitive anti-inflammatory agents, such as IL10 and/or a soluble TNF receptor and/or trefoil factor via the oral route. The preferred feature according to the invention is the inoculation with a suspension of live recombinant yeast cells, preferably *Saccharomyces* cells which have been engineered to produce the respective proteins. As example, mice were used in which a chronic inflammation of the distal colon had been induced by administration with dextran sulfate sodium (DSS). The treatment as scored by histological evaluation clearly resulted in a regression of the inflammation and disease symptoms. The finding is highly unexpected since, as it was assumed that only living lactic acid bacteria, capable of interacting with the intestinal mucosa were suitable for delivering cytokines and curing colitis.

The immune system in a mammal is diverse and complex and includes natural and adaptive immune mechanisms and reactions. The immune system is often described in terms of either humoral or cellular immune responses. Humoral immunity refers broadly to antibody production and actions by B-cells; cellular immunity is mediated by cells including T-cells, dendritic cells, neutrophiles, monocytes and macrophages. T-cells and B-cells are two categories of lymphocytes.

One of the mechanisms by which the immune system normally acts and regulates itself includes the production of so-called cytokines. It is known that cytokines mediate several positive and negative immune responses. Cytokines normally act by binding to a receptor on a target cell. The activity of cytokines can be interfered with in several ways, for example by administration of soluble receptors (extracellular domains of the receptor) or by cytokine analogues or derivatives.

IL-10 is a cytokine capable of mediating a number of actions or effects. It is known that IL-10 is involved in controlling the immune responses of different classes or subsets of Th cells (T-helper cells).

Inflammatory bowel disease (IBD) refers to a group of gastrointestinal disorders characterized by a chronic non-specific inflammation of portions of the gastrointestinal tract. Ulcerative colitis (UC) and Crohn's Disease (CD) are the most prominent examples of IBD in humans. They are associated with many symptoms and complications, including growth retardation in children, rectal prolapse, blood in stools (e.g. melena and/or hematochezia), wasting, iron deficiency, and anemia, e.g. iron deficiency anemia and anemia of chronic disease or of chronic inflammation. The etiology or etiologies of IBD are unclear. Reference hereto is made in Wyngaarden and Smith (eds.) Cecil's Textbook of Medicine (W.B. Saunders Co. 1985), Berkow (ed.) The Merck Manual of Diagnosis and Therapy (Merck Sharp & Dohme Research Laboratories, 1982), and Harrison's Principles of Internal Medicine, 12th Ed., McGraw-Hill, Inc. (1991).

The incidence of IBD varies greatly with geographic location. A collaborative study over Europe shows an incidence per 100 000 of 10,4 for UC and of 5,6 for CD with 40% respectively 80% higher incidence for UC and CD in northern centres when compared to those in the south. As both UC and CD are long time affections, they correspond to real disturbances in the quality of life. Crohn's disease has a bimodal age distribution of onset showing striking peaks in incidence at 20 and 50 years of age. A higher incidence and more grievous disease profile is associated with the peak at younger age. This makes CD especially poignant as afflicted adolescents and young adults are virtually deprived form the high expectations form life, so particularly associated with this social group.

Ulcerative colitis refers to a chronic, non-specific, inflammatory, and ulcerative disease having manifestations primarily in the colonic mucosa. It is frequently characterized by bloody diarrhea, abdominal cramps, blood and mucus in the stools, malaise, fever, anemia, anorexia, weight loss, leukocytosis, hypoalbuminemia, and an elevated erythrocyte sedimentation rate (ESR).

Complications can include hemorrhage, toxic colitis, toxic megacolon, occasional rectovaginal fistulas, and an increased risk for the development of colon cancer.

Ulcerative colitis is also associated with complications distant from the colon, such as arthritis, ankylosing spondylitis, sacroileitis, posterior uveitis, erythema nodosum, pyoderma gangrenosum, and episcleritis.

Treatment varies considerably with the severity and duration of the disease. For instance, fluid therapy to prevent dehydration and electrolyte imbalance is frequently indicated in a severe attack. Additionally, special dietary measures are sometimes useful. Medications include various corticosteroids, sulphasalazine and some of its derivatives, and possibly immunosuppressive drugs.

Crohn's Disease shares many features in common with ulcerative colitis. Crohn's Disease is distinguishable in that lesions tend to be sharply demarcated from adjacent normal bowel, in contrast to the lesions of ulcerative colitis which are fairly diffuse. Additionally, Crohn's Disease predominately afflicts the ileum (ileitis) and the ileum and colon (ileocolitis). In some cases, the colon alone is diseased (granulomatous colitis) and sometimes the entire small bowel is involved (jejunoileitis). In rare cases, the stomach, duodenum, or oesophagus are involved. Lesions include a sarcoid-type epithelioid granuloma in roughly half of the clinical cases. Lesions of Crohn's Disease can be transmural including deep ulceration, edema, and fibrosis, which can lead to obstruction and fistula formation as well as abcess formation. This contrasts with ulcerative colitis which usually yields much shallower lesions, although occasionally the complications of fibrosis, obstruction, fistula formation, and abcesses are seen in ulcerative colitis as well.

Treatment is similar for both diseases and includes steroids, sulphasalazine and its derivatives, and immunosuppressive drugs such as cyclosporin A, mercaptopurine and azathioprine. More recently developed treatments, some still in clinical trials, involve systemic administration (by injection) of TNF blocking compounds such as TNF-antibodies. The publication Digestive Diseases and Sciences, vol. 45, 2000, 1462-4 discloses the ability of the yeast Saccharomyces boulardii together with mesalamine to reduce the incidence of clinical relapse in Crohn's Disease patients.

IBD represents a genuine problem in public health because of the absence of etiologic treatment. Although many patients are managed successfully with conventional medical therapy, such as anti-inflammatory corticosteroid treatment, most will have recurrent activity of disease and two-thirds will require surgery.

The cause of inflammatory bowel diseases is unknown. The pathogenesis of CD and UC probably involves interaction between genetic and environmental factors, such as bacterial agents, although no definite etiological agent has been identified so far. The main theory is that abnormal immune response, possibly driven by intestinal microflora, occurs in IBD. However, what is well established is that T-cells play an important role in the pathogenesis. Activated T-cells can produce both anti-inflammatory and pro-inflammatory cytokines. With this knowledge in hand, IBD can be counteracted in a rational manner. Novel anti-inflammatory therapies, which make use of neutralising monoclonal antibodies or anti-inflammatory cytokines, show the possibility to modulate the immune disregulations causative to IBD. A highly prominent and effective new therapy is systemic treatment with anti-TNF monoclonal antibodies as mentioned above. Single intravenous doses, ranging from 5 to 20 mg.kg⁻¹, of the cA2 infliximab antibody resulted in a drastic clinical improvement in active Crohn's disease. The use of systemically administered recombinant IL-10 in a 7 day by day treatment regime using doses ranging from 0.5 to 25 µg.kg⁻¹ showed reduced Crohn's disease activity scores and increased remission. A number of very promising therapies, either tangling pro-inflammatory cytokines or the establishment of T cell infiltrates, are currently emerging from experimental models. All these strategies however require systemic administration. The severe complications of IBD can be seriously debilitating, and eventually may lead to death.

Several methods to treat IBD are known in the art. In US Patent 5,368,854, assigned to Schering Corp., a method is disclosed using IL-10 to treat inflammatory bowel diseases in mammals. In this method the cytokine is administered to a mammal having an IBD (inflammatory bowel disease). The administration of IL-10 as described in this reference is parenteral such as intravascular and preferably intravenous.

It is obvious however that such a route of administration for a (human) patient suffering from an IBD is not without drawbacks. A much easier and more convenient way is an oral administration of a medicament comprising a cytokine such as IL-10 or a cytokine-antagonist which has a similar therapeutic activity. More importantly, localized release of the therapeutic agent allows for higher efficacy and less unwanted side effects due to systemic activities.

In WO 97/14806, assigned to Cambridge University Technical Services Ltd., a method is disclosed for delivering biologically active polypeptides and/or antigens by using non-invasive bacteria, such as *Lactococcus,* by intranasal administration of said polypeptides in the body, especially at the mucosa. WO 00/23471 is teaching in detail how colitis can be treated by a cytokine-producing *Lactococcus* strain.

In order to achieve the recovery of a patient suffering from an IBD, it is necessary to restore the damaged cells and the organ comprising said damaged cells, for instance the colon. Several studies indicate that the healing effect of the anti-inflammatory compound producing *Lactococcus* is due to the combination of living lactic acid bacteria and the anti-inflammatory compound, and not to the anti-inflammatory compound alone. Indeed, both Steidler *et* al.(2000), for IL10, and Vandenbroucke *et al.* (2004) in case of trefoil factors, clearly demonstrate that no effect was obtained when the IL10 producing *Lactococcus* is killed by UV irradiation before treatment, although in these cases active compound should be released in the intestine by the lysing bacteria.

Treatment of colitis with IL-10 producing *Lactococcus lactis* has proven to be successful. The major drawback however is the rapidly decreasing viability of *Lactococcus,* and the poor survival in the intestine, making an accurate dosing and timing of the treatment difficult.

As an alternative for the delivery of proteins or peptides in the gut, Blanquet et al. (2004, WO 01/98461) describe the possibility to deliver proteins or peptides in the gut, using recombinant *Saccharomyces cerevisiae.* Although this system may be useful in some cases, it is generally accepted that this way of delivery might not be successful for diseases where an interaction with the intestinal mucosa is necessary. Indeed, as the authors used an artificial gut system to test the delivery, they indicated themselves that "this system can not simulate the physiological processes of the gut wall, such as active and facilitated transport." No data about the bioavailability or activity of the active molecules are available (Blanquet *et al.* 2004). It is well documented that, especially in the case of IL-10, the local cytokine microenvironment produced by the gene-secreting cell types are extremely important (Croxford *et al.,* 2001). Several factors may play a role, including, but not limited to the cell wall composition of the cytokine producing cell, the cell size, and the capability of the cell to interact with the mucosa. Moreover, there are recent data that yeast metabolic products, yeast antigens and yeasts are possible triggers for irritable bowel syndrome (Santelmann and Howard, 2005). Therefore, the results obtained with lactic acid bacteria cannot be extrapolated to yeast without undue experimentation, and in view of the special immunomodulating characteristics of *Lactococcus,* the person skilled in the art would not expect a positive result of yeast, delivering in situ in the gut an anti-inflammatory agent such as IL10, for the treatment of colitis.

Surprisingly we found that recombinant yeast, producing an anti-inflammatory compound such as IL10 can be used for the treatment of mucosal inflammation, such as IBD or mucositis.

The following is our invention:
1. Use of a recombinant anti-inflammatory compound producing yeast strain and/or a cytokine antagonist-producing yeast strain for the preparation of a medicament to treat mucosal inflammation, whereby said anti-inflammatory compound or cytokine antagonist is mutated to avoid or limit glycosylation.
2. The use of a recombinant yeast according to 1, whereby said mucosal inflammation is an inflammatory bowel disease.
3. The use of a recombinant yeast strain according to 1 or 2 wherein the anti-inflammatory compound or cytokine antagonist is selected from the list consisting of IL-10, a trefoil factor such as TFF1, TFF2 or TFF3, a TNF antagonist such as the soluble TNF receptor, INCA, ABIN, an IL-12 antagonist, an Interferon-γ antagonist, an IL-1 antagonist and a virus-coded cytokine analogue such as EBV BCRF1.
4. The use of a recombinant yeast according to any of the preceding items, whereby said anti-inflammatory compound is non-glycosylated IL-10.
5. The use of a recombinant yeast strain according to any of the preceding items wherein the yeast strain is selected from the group consisting of Saccharomyces sp., Hansenula sp., Kluyveromyces sp. Schizzosaccharomyces sp. Zygosaccharomyces sp., Pichia sp., Monascus sp., Geotrichum sp and Yarrowia sp.
6. Use of a recombinant yeast strain according to 5 wherein the Saccaromyces sp is Saccharomyces cerevisiae.
7. Use of a recombinant yeast strain according to 6 wherein the recombinant yeast strain is Saccharomyces cerevisiae subspecies boulardii.
8. Use of a recombinant yeast strain according to any of the preceding items wherein the inflammatory bowel disease is a chronic colitis, Crohn's disease or an ulcerative colitis.

The anti-inflammatory compound to be produced by the yeast host strain can be any anti-inflammatory compound known to the person skilled in the art. Alternatively, the yeast may produce a modifying enzyme, such as, as a non-limiting example, a kinase, a phosphatase, a protease or an acetylating enzyme, which may convert an inactive substrate into an anti-inflammatory compound. As a non-limiting example, said anti-inflammatory compound may be a cytokine, such as IL-10, a cytokine antagonist (such as a TNF antagonist, an IL-12 antagonist, an interferon-y antagonist, or an IL-1 antagonist), an anti-inflammatory polypeptide (such as trefoil, ABIN or INCA proteins), or a virus-coded cytokine analogue such as EBV BCRF1. Cytokine antagonists are known to the person skilled in the art and include, but are not limited to the soluble receptor, and anti-cytokine antibodies. Preferably, said anti-inflammatory compound is IL-10.

In case that anti-inflammatory compound comprises glycosylation sites, preferably one or more of these sites are mutated to avoid or limit glycosylation, preferably to avoid or limit hyperglycosylation in yeast. A preferred embodiment is a recombinant yeast, producing a non-glycosylated IL-10, preferably a non-glycosylated IL-10 in which one or more possible glycosylation sites have been mutated.

Preferably, the gene or genes encoding the anti-inflammatory compound are heterologous genes, situated on a plasmid. Suitable plasmids are known to the person skilled in the art and include but are not limited to episomal plasmids, artificial chromosomes or integrative plasmids.

The recombinant yeast may be any yeast capable of surviving in the mammalian intestine. Preferably, said yeast has a known probiotic capacity, such as yeast strains selected from kefir, kombucha or dairy products. Even more preferably, said recombinant yeast is selected from the group consisting of *Saccharomyces* sp., *Hansenula* sp., *Kluyveromyces* sp. *Schizzosaccharomyces* sp. *Zygosaccharomyces* sp., *Pichia* sp., *Monascus* sp., *Geotrichum* sp and *Yarrowia* sp. Still more preferably, said yeast is *Saccharomyces cerevisiae,* most preferably *Saccharomyces cerevisiae* subspecies *boulardii.* Preferably, the recombinant yeast host - vector system is a biologically contained system. Biological containment is known to the person skilled in the art and can be realized by the introduction of an auxotrophic mutation, preferably a suicidal auxotrophic mutation such as the Thy A mutation, or its equivalents. Alternatively, the biological containment can be realised at the level of the plasmid carrying the gene encoding the anti-inflammatory compound. This can be realized, as a non-limiting example, by using an unstable episomal construct, which is lost after a few generations.

Several levels of containment, such as plasmid instability and auxotrophy, can be combined to ensure a high level of containment

Preferably, the mucosal inflammation is IBD. Inflammatory bowel diseases such as a chronic colitis, Crohn's disease or an ulcerative colitis can be treated according to the invention with an appropriate dosage of the active anti-inflammatory compound, preferably IL-10, even more preferably non-glycosylated IL-10 and provides unexpectedly a restoration of the diseased colon to an apparently normal and healthy state.

Alternatively the administration of an anti-inflammatory compound such as IL-10 by recombinant yeast can be used to treat other mucosal inflammations such as mucositis.

IL-10 can be administered alone or in combination with at least one additional therapeutic agent. Examples of such agents include corticosteroids; sulphasalazine, derivatives of sulphasalazine, immunosuppresive drugs such as cyclosporin A, mercaptopurine, azathioprine, and another cytokine. The co-administration can be sequential or simultaneous. Co-administration generally means that the multiple (two or more) therapeutics are present in the recipient during a specified time interval. Typically, if a second agent is administered within the half-life of the first agent, the two agents are considered co-administered. The other therapeutic agent as mentioned here may be another microbial delivery system such as *Lactococcus* or a yeast strain, delivering another compound, preferably a compound with a complementary action such as, as a non limiting example, trefoil factor.

The invention disclosed herein thus concerns a localised delivery of IL-10 through in situ synthesis by recombinant yeast. As a result thereof the inflammation is reduced by at least 30%, in chronic colitis induced with DSS and prevents the onset of colitis in IL-10 -/- 129 Sv/Ev mice. So the method is equally efficient in comparison to powerful, well-established and accepted therapies relying on the systemic administration of anti-inflammatory proteins.

The yeast vector used here is selected from food, preferably food with probiotic characteristics, or from known probiotics, and is totally harmless for immunocompetent individuals. Especially in the case of *Saccharomyces cerevisiae* subspecies *boulardii,* clinical experience is available, and the transit time in the intestine has been studied. Accurate dosage and timing during treatment, shown here to be of great importance, can thus easily be obtained.

The critical requirement for viability of the vector is shown in the current invention. This indicates the need for in situ synthesis of IL-10. The vector is indeed capable to achieve this by showing de novo synthesis of IL-10 in the colon. Yeast, according to the invention has in this respect a clear advantage above *Lactococcus lactis* as described in WO 00/23471, as it keeps it viability easier during processing, and it is surviving better in the intestine than is *Lactococcus.*

This method may answer the question for sustained and localised presence of IL-10 in therapy at concentrations higher than desirable or even achievable through systemic delivery, with regard to latent side effects.

### DEFINITIONS

Some terms used in the current description are, for sake of clarity, explained hereafter. Generally, the term *"symptoms"* refers to any subjective evidence of disease or of a patient's condition. This includes evidence as perceived by the patient. Examples of symptoms of IBD include diarrhea, abdominal pain, fever, melena, hematochezia, and weight loss.

The term *"signs"* refers generally to any objective evidence of a disease or condition, usually as perceived by an examining physician or features which would reveal themselves on a laboratory evaluation or other tests such as an ultrasonic study or a radiographic test. Some examples of signs of IBD include abdominal mass, glossitis, aphtous ulcer, anal fissure, perianal fistula, anemia, malabsorption, and iron deficiency. Occasionally, signs and symptoms overlap. For example, the patient complains of blood stools (a symptom), and a laboratory test of a stool sample is positive for blood (a sign).

The phrase *"appropriate dosage"* or *"effective amount"* means an amount or dosage sufficient to ameliorate a symptom or sign of an autoimmune condition or of an undesirable or inappropriate inflammatory or immune response. An effective amount for a particular patient may vary depending on factors such as the condition being treated, the overall health of the patient, the method route and dose of administration and the severity of the side affects.

With *"cytokine"* is meant a polypeptide factor produced transiently by a range of cell types, acting usually locally, and activating the expression of specific genes by binding to cell surface receptors.

With *"antagonist"* is meant a compound that binds to but does not activate receptors, hence does inhibit the action of an agonist competitively.

*"Agonists"* are compounds that bind to and activate receptors (e.g., endogenous ligands such as hormones and neurotransmitters, chemically synthesized compounds, natural products like alkaloids).

*"Compound"* means any chemical of biological compound, including simple or complex organic and inorganic molecules, peptides, peptido-mimetics, proteins, antibodies, carbohydrates, nucleic acids or derivatives thereof.

The terms *"protein"* and *"polypeptide"* as used in this application are interchangeable. *"Polypeptide"* refers to a polymer of amino acids and does not refer to a specific length of the molecule. This term also includes post-translational modifications of the polypeptide, such as glycosylation, phosphorylation and acetylation

### BRIEF DESCRIPTION OF THE FIGURES

Figure 1: Plasmid map of pPIC92MIL10
Figure 2: Plasmid map of the vector pYES2
Figure 3: Plasmid map of pYES2-mIL10
Figure 4: Construction of the TPI-ppMF-mIL10 fragment by overlay PCR. The numbers indicate the primers used (see text).
Figure 5: Plasmid map of pYES2T-mIL10
Figure 6: mIL10 secretion by *Saccharomyces cerevisiae* INV S.c.1 [pYES2] and *Saccharomyces cerevisiae* INV S.c.1 [pYES2T-mIL10] as determined by sandwich ELISA in function of time (after 0, 8, 12, 24 and 48 hour).
Figure 7: Western blot detection of mIL10. The signal corresponds to the amount of protein present in 0.5 ml culture supernatant after 24 hours of growth in YPD.
Figure 8: Western blot detection of mIL10 and non-glycosylated mIL-10. The signal corresponds to the amount of protein present in 0.5 ml culture supernatant after 24 hours of growth in YPD.

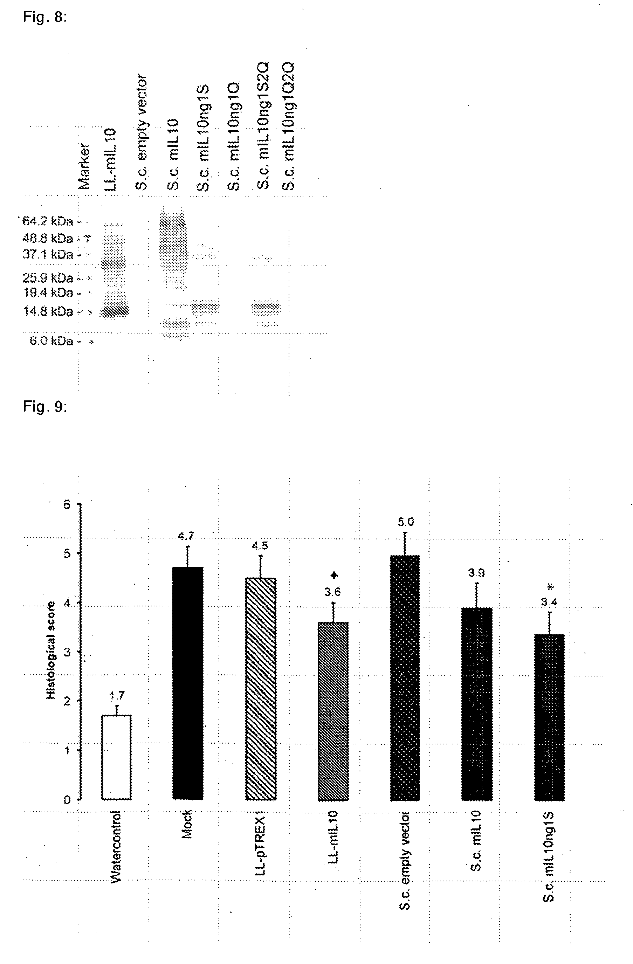

Figure 9: Statistical evaluation of the histological score of the distal colon. All data are expressed as mean ± SEM. Data were statistically analyzed with a 1-way analysis of variance (ANOVA) followed by a Fisher's least significant difference (LSD) multiple comparisons posttest. * represent a statistically significant difference in comparison with mock and *Saccharomyces cerevisiae* vector control treated groups of *P* < 0.035 and P < 0.012, respectively ⁺a represent a statistical difference in comparison with mock and *Lactococcus lactis* vector control treated groups of *P* < 0.085 and P < 0.152, respectively.
Figure 10: Gene replacement of P_{URA3}-URA3 by P_{TPI}-ppMF-mIL10ng1S in *Saccharomyces cerevisiae VC5.* The numbers (1-8) indicate the different primers used.
Figure 11: Genomic organization of the 108 504 - 120 299 bp region of chromosome V of *Saccharomyces cerevisiae* (Dietrich *et al.,* 1997); YEL023C en YEL020C are genes with an unknown function; GEA2 codes for guanine nucleotide exchange factor 2 of the ADP ribosylation factor (ARF); URA3 codes for orotidine-5'-phosphate (OMP) decarboxylase; TIM9 codes for the mitochondrial inter membrane protein that is responsible for the import and insertion of polytopic inner membrane proteins.
Figure 12: Western blot detection of mIL10. The signal corresponds to the amount of protein present in 0.5 ml culture supernatant after 24 hours of growth in YPD.

### EXAMPLES

### Materials and methods to the examples

### Strains and Culture media

*Saccharomyces cerevisiae* INV Sc1 (mating-α, his3Δ1, leu2-3,-112 trp1-289 and ura3-52) was obtained from Invitrogen™.

*Saccharomyces cerevisiae* subspecies *boulardii* was isolated from a commercial probiotic preparation.

Minimal medium was SD+CSM-U, consisting of 0,67% Yeast Nitrogen Base w/o Amino Acids (Difco, Detroit, MI) 2 % dextrose (Merck, Darmstadt, Germany) and 0.077% CSM-URA (Bio101 Systems, Morgan Irvine, CA).

YPD medium consists of 1% yeast extract, Difco; 2% dextrose, Merck; 2% peptone, Difco.

### Recombinant DNA techniques.

PCR amplification of DNA was performed with VENT polymerase and using conditions recommended by the manufacturer. DNA modifying enzymes and restriction endonucleases were used under standard conditions and in the buffers recommended by the manufacturers. General molecular cloning techniques and the electrophoresis of DNA and proteins were carried out essentially as described (Sambrook et al., 1990). *S. cerevisiae* was transformed by electroporation and transformants were selected on the suitable selective medium as indicated

### Construction of the expression plasmids.

### Subcloning of mIL10 in the plasmid pPIC92

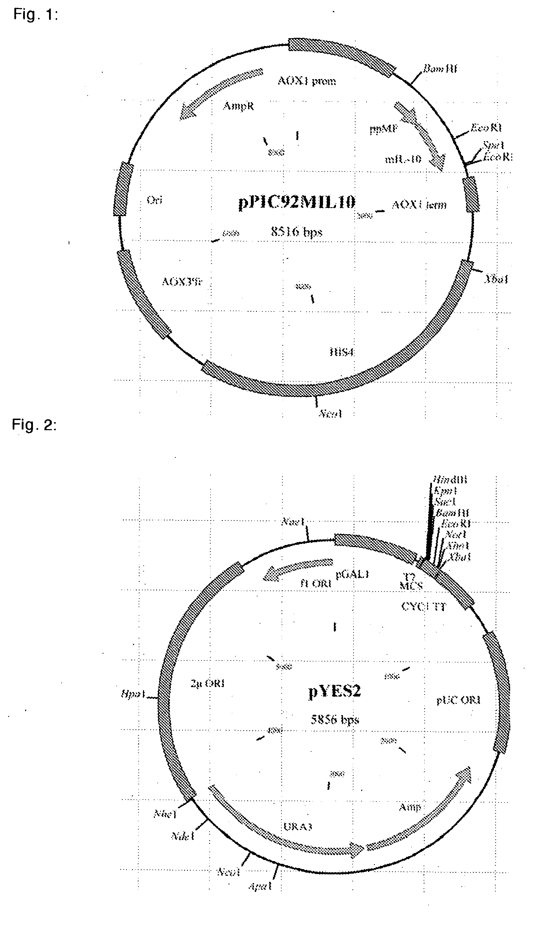

The DNA coding sequence of mature mIL10 was PCR amplified (Vent® polymerase, NEB, Ipswich, MA) with oligo mIL10 S (CAGTACAGCCGGGAAGACAAT) and oligo mIL10 AS (GCACTAGTTAGCTTTTCATTTTGAT) from the plasmid pT1mIL10 (Schotte *et al.,* 2000). This resulted in a DNA fragment of 474 bp. This mIL10 PCR fragment was ligated in frame after the prepro α-mating factor (ppMF) secretion signal of *Saccharomyces cerevisiae* present on the plasmid pPIC92 that was linearized with the restriction enzyme Nael (NEB). The resulting construct was designated pPIC92mIL10 (Figure 1). The plasmid pPIC92 is derived form the plasmid pPIC9K (Invitrogen™, Carlsbad, CA). *Escherischia coli* MC1061 heat competent cells were transformed with the pPIC92mIL10 ligation mix.

### Subcloning of ppMF-mIL10 into plasmid pYES2

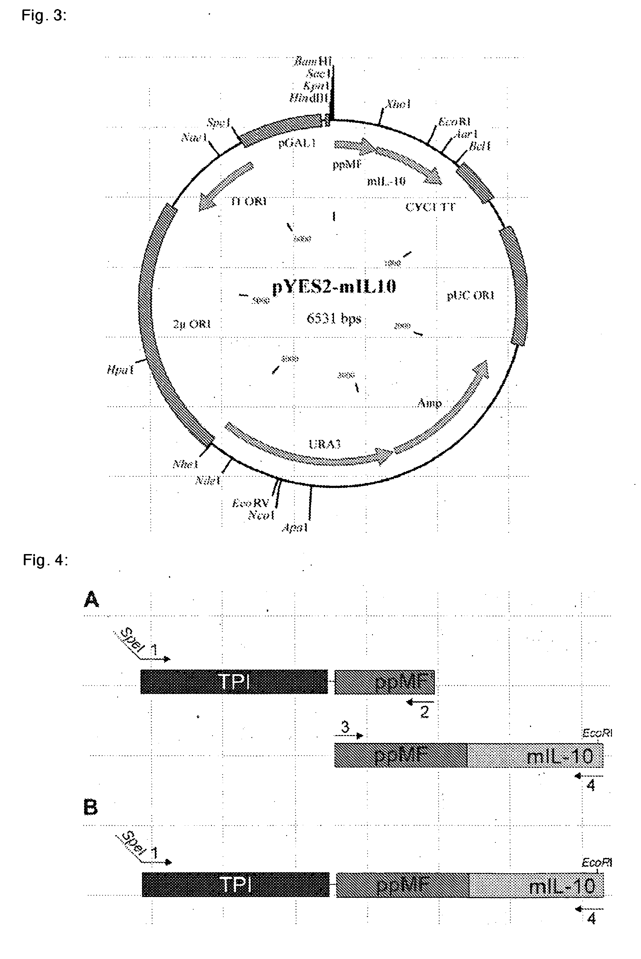

pYES2 (Figure 2; Invitrogen) is a 5.9 kb vector designed for inducible expression of recombinant proteins in *Saccharomyces cerevisiae.* Features of the vector allow easy cloning of your gene of interest and selection of transformants by uracil prototrophy (it contains the URA3 marker). The pYES2 vector is designed for high-level expression of recombinant proteins in *Saccharomyces cerevisiae.* It contains the GAL1 promotor that allows galactose inducible expression. The vector carries the 2µ origin of replication and is maintained episomally in high copy (10-40 copies per cell). The vector contains also the *E. coli* pUC origin and ampicillin resistance that allows easy cloning and selection in *E. coli.*
The vector pYES2 was digested with a combined digest of *Bam*HI (NEB)and *Xba*I (NEB). The vector DNA fragment of 5780 bp was isolated. The plasmid pPIC92mIL10 was digested with a combined digest of *Bam*HI (NEB) and *Spe*l (NEB). The DNA fragment of 751 bp was isolated. The two selected DNA bands (5780 and 751 bp were ligated and transformed to *E*. *coli* MC1061 heat competent cells. The constructed plasmid was designated pYES2-mIL10 (Figure 3).

### Construction of the plasmid pYES2T-mIL10

The previously constructed plasmid pYES2-mIL10 had the disadvantage that there is only mIL10 secretion upon induction with galactose of the GAL1 promotor. For *in vivo* use of Saccharomyces strains that secrete mIL10, it is very important that they constitutively secrete mIL10. To accomplish this goal, we replaced the GAL1 promotor by the constitutive and very strong triose phosphate isomerase (TPI promotor). The ppMF-mIL10 expression cassette was subcloned under control of the constitutive and strong TPI promotor on a *Saccharomyces cerevisiae* high copy number (2µ origin) plasmid.

The TPI-ppMF' fragment was PCR amplified with oligo Spel-TPI-S (oligo No° 1 on Figure 4; GCACTAGTATCCGAGATTATATCTAGGAACCCATCAGG) and antisense oligo ppMF-middle-AS (oligo No° 2 on Figure 4A; CTTCTAAATCTGAGTAACCGATGACAGCTTC) from the plasmid pSCTPIMF3. This resulted in the TPI-ppMF' PCR which has a length of 500 bp.

The ppMF-mIL10 PCR fragment was amplified with the oligo ppMF-start-S (oligo No° 3 on Figure 4A; ATGAGATTTCCTTCAATTTTTACTGCAG) ant oligo mIL10-*Eco*RI-middle-AS (oligo No° 4 on Figure 4A; CAGGGAATTCAAATGCTCCTTGATTTCTGG) form the previously constructed plasmid pPIC92mIL10. The resulting PCR fragment ppMF-mIL10 had a length of 525 bp.

The TPI-ppMF' and ppMF-mIL10 fragment were used as template in an overlay PCR with the outer oligo's from the two previous PCR reactions: oligo *Spe*l-TPI-S (oligo No° 1 on Figure 4B; GCACTAGTATCCGAGATTATATCTAGGAACCCATCAGG) and oligo mIL10-*Eco*RI-middle-AS (oligo No° 4 on Figure 4*B*; CAGGGAATTCAAATGCTCCTTGATTTCTGG). The assembled *Spe*I-TPI-ppMF-mIL10-*Eco*RI PCR fragment (Figure 4*B*) had a length of 989 bp en was purified on an agarosegel and digested by the restriction enzymes Spel and *Eco*RI.

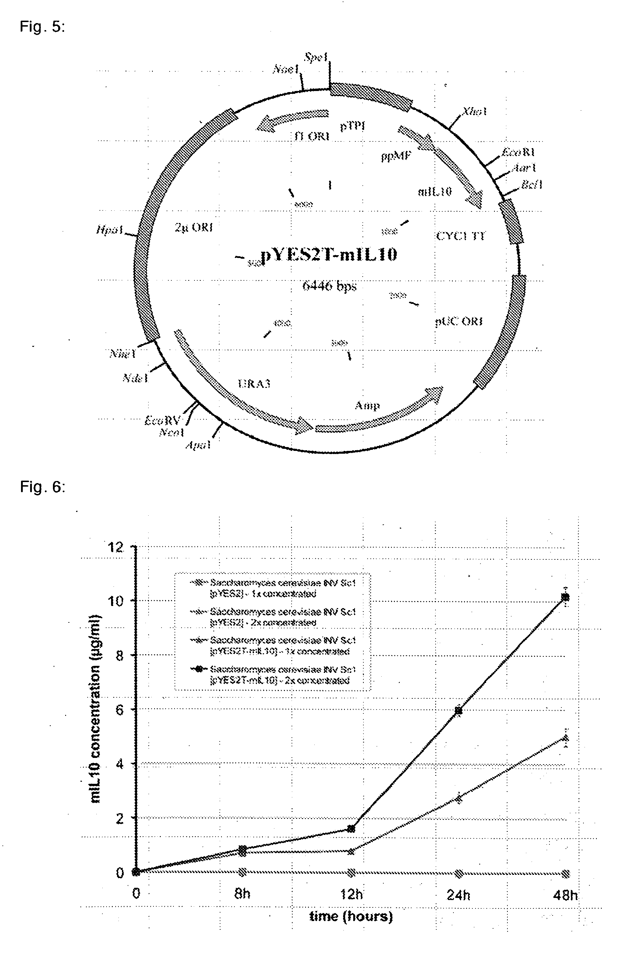

The previously constructed vector pYES2-mIL10 was digested by *Spe*I and *Eco*RI*.* The DNA fragment of 5466 bp was isolated and ligated with the *SpeI-*TPI-ppMF-mIL10-*Eco*RI PCR fragment. This resulted in a plasmid that was designated pYES2T-mIL10 (Figure 5). Heat competent MC1061 *E. coli* cells were transformed with the pYES2T-mIL10 ligation mixture.

### Animals

11-week old female BALB/c mice were obtained form Charles River Laboratories (Sulzfeld, Germany). They were housed under SPF conditions. All mice were fed standard laboratory feed and tap water ad libitum. The animal studies were approved by the Ethics Committee of the Department for Molecular Biomedical Research, Ghent University (File No. 04/02).

### Induction of chronic colitis by DSS

Mice weighing approximately 21 g were induced to chronic colitis by four cycles of administration of 5% (w/v) DSS (40 kDa, Applichem, Darmstadt, Germany) in the drinking water, alternating with 10-day periods of recovery with normal drinking water. (Okayasu *et al.,* 1990; Kojouharoff *et al.,* 1997) Treatment was arbitrarily initiated at day 21 after the fourth cycle of DSS. The different groups were treated for 14 days. 14 days after the last treatment, mice were killed and analyzed.

### Statistical analysis

All data are expressed as mean ± SEM. Data were statistically analyzed with a 1-way analysis of variance (ANOVA) followed by a Fisher's least significant difference (LSD) multiple comparisons posttest.

### Lactococcus control

Treatment with IL-10 secreting *Lactococcus* was carried out as described by Steidler *et al.* (2000)

### Example 1: Construction of mIL10 secreting Saccharomyces strains

1 µg of the plasmid pYES2T-mIL10 (prepared by Qiagen midi plasmid kit, Hilden, Germany; out of the *E. coli* strain MC1061[pYES2T-mIL10]) was electroporated into electrocompetent *Saccharomyces cerevisiae* INV Sc1 cells. The transformed yeast cells were plated out on uracil deficient (selection) minimal medium. PCR screening identified *Saccharomyces cerevisiae* transformants in which the pYES2T-mIL10 plasmid was present. These were designated *Saccharomyces cerevisiae* INV Sc1[pYES2T-mIL10].

### Example 2: mIL10 secretion by Saccharomyces cerevisiae.

One colony of the Saccharomyces strains *Saccharomyces cerevisiae* INV Sc1[pYES2T-mIL10] and the vector control *Saccharomyces cerevisiae* INV Sc1[pYES2] were respectively inoculated in 50 ml minimal uracil deficient medium (SD+CSM-U). After 24 hours of aerobic growth at 30°C the cells were pelleted by centrifugation (5 minutes@2500 tmp) and unconcentrated (2x 10E⁸ CFU/ml) or 2 x concentrated in YPD medium. At different time points (8, 12, 24 and 48 hours), supernatant samples were taken for mIL10 quantification and characterization. During the 48 hours of growth, pH of the *Saccharomyces cerevisiae* supernatant remained stable at 7.

A sandwich ELISA was set up to assess the amount of murine IL-10 secreted in the culture supernatant. Polyclonal rabbit anti murine IL-10 (5 µg ml⁻¹; Prepro Tech, London, England) was used as capture antibody. A monoclonal, biotin-coupled antibody against murine IL-10 (Pharmingen, San Diego, USA) was applied at a 1/1000 dilution to detect the captured IL-10. The biotinylated complexes were reacted with horseradish peroxidase-coupled streptavidin (Pharmingen) at a 1/1000 dilution and revealed by reaction with TMB substrate (Pharmingen). Between steps the microtitre plates were washed twice with water and once with PBS, containing 0.05% Triton X-100 (Sigma). In order to prevent non-specific binding the plates were incubated in PBS containing 0.1 % casein.

After 12 hours of growth, the *Saccharomyces cerevisiae* INV Sc1 [pYES2T-mIL10] strain had secreted 0.8 ± 0.0 µg/ml mIL10 when the cells were 1 x concentrated and 1.6 ± 0.1 µg/ml mIL10 when the cells were 2 x concentrated (Figure 6). After 24 hours of growth, the *Saccharomyces cerevisiae* INV Sc1 [pYES2T-mIL10] strain had secreted 2.8 ± 0.2 µg/ml mIL10 when the cells were 1 x concentrated and 6.0 ± 0.2 µg/ml mIL10 when the cells were 2 x concentrated (Figure 6). And finally, after 48 hours of growth, the *Saccharomyces cerevisiae* INV Sc1 [pYES2T-mIL10] strain had secreted 5.0 ± 0.3 µg/ml mIL10 when the cells were 1 x concentrated and 10.2 ± 0.3 µg/ml mIL10 when the cells were 2 x concentrated (Figure 6). The empty vector control *Saccharomyces cerevisiae* INV Sc1 [pYES2] showed no mIL10 production at any given time point (Figure 6).

Proteins were extracted from the culture supernatant by TCA precipitation and subsequently dissolved in Laemmli sample buffer (Laemmli, 1970). Protein fractions were separated by sodium dodecyl sulphate polyacrylamide gel electrophoresis (PAGE) (SDS-PAGE) and electroblotted onto a nitrocellulose membrane (Burnette, 1981).

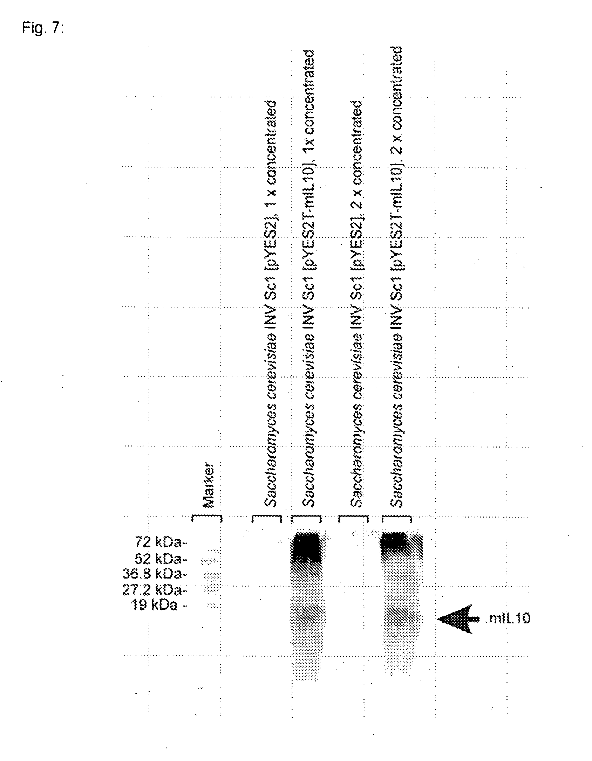

Murine interleukin-10 was detected by immunoblotting with polyclonal rabbit anti murine IL-10 as the primary antibody at a 1/1000 dilution (Prepro Tech, London, U.K.). The secondary antibody was goat anti rabbit IgG (H+L) coupled to alkaline phosphatase (SBA, Birmingham, USA) and was used at a 1/1000 dilution. Enzymatic activity was revealed with NBT/BCIP substrate (Boehringer Mannheim, GmbH, Germany). Figure 7 shows mIL10 detection in the *Saccharomyces cerevisiae* INV S.c.1 supernatant by Western blot after 24 hours of growth. Saccharomyces cells were 1 x or 2 x respectively concentrated in YPD for the 24 hours of growth.

The biologic activity of the recombinant IL-10, secreted by *Saccharomyces,* was tested in a proliferation assay with the MC/9 mouse mast cell line. The biologic titer of IL-10 was determined from the stimulation of incorporation of [³H]thymidine by the proliferating mast cells. A standard of known specific activity (BioSource International, Camarillo, CA) was used as an internal control.

### Example 3: Production of non-glycosylated murine IL10

As glycosylation may affect the activity of the secreted IL-10, we wanted to generate a *Saccharomyces cerevisiae* strain that secretes non-glycosylated murine Interleukin-10. mIL10 contains two potential *Saccharomyces cerevisiae* N-glycosylation sites (N-X-S/T consensus sequence, potential glycosylation sites are indicated in bold):

The site 11-NCT-19 is located in a loop that is orientated towards the solvent. The site does not appear to be involved in the interaction with the IL-10 receptor. This recognition site is not conserved in human (h)IL10. In hIL10, the amino acid sequence is 11-SCT-13. The 11-NCT-13 site seems to by an ideal glycosylation site for *Saccharomyces cerevisiae.* This glycosylation site can be removed from mIL10 by mutating the 11-NCT-13 to 11-SCT-13 like in the hIL10 or to 11-QCT-13 (Q is an amino acids that structurally closely resembles N; mutations are indicated in bold). Both mutations of mIL10 were made.

The site 116-NKS-118 seems to be important for the stabilization and structure of mIL10. N as wells as S are involved in H-bound formation with the backbone of nearby residues. The amino acid sequence of this site is strictly conserved in human and all other known homologues of IL10. The program GlyProt does not recognize this site as a potential glycosylation site. In hIL10 this site is conserved and is not glycosylated (Vieira et al., 1991). This suggests that the 116-NKS-118 site is also not glycosylated in murine IL10. Nevertheless, the mutation of the 116-NKS-118 site into 116-QKS-118 was made to test possible effects.

In total, four different *Saccharomyces cerevisiae* constructs were made that secrete 4 different mutant (non-glycosylated) forms of mIL10. Two constructs are mutated in the first glycosylation site which is mutated to respectively S and Q. In addition we also made 2 constructs in which the first glycosylation site was mutated to respectively S and Q and the second glycosylation site is mutated to Q.

In the mIL10ng 1S mutant only the first potential glycosylation site is mutated. The 11-NCT-13 sequence is mutated to 11-SCT-13.

In the mIL 10ng1Q mutant only the first potential glycosylation site is mutated. The 11-NCT-13 sequence is mutated to 11-QCT-13

In the mIL10ng1S2Q mutant the first (11-NCT-13) and the second (116-NKS-118) potential glycosylation sites are both mutated. The first potential glycosylation site 11-NCT-13 is mutated to 11-SCT-13. The second potential glycosylation site 116-NKS-118 is mutated to 116-QKS-118

In the mIL10ng1Q2Q mutant the first (11-NCT-13) and the second (116-QKS-118) potential glycosylation sites are mutated. The first potential glycosylation site 11-NCT-13 is mutated to 11-QCT-13. The second potential glycosylation site 116-NKS-118 is mutated to 116-QKS-118. The mIL10ng1S, mIL10ng1Q, mIL10ng1S2Q and mIL10ng1Q2Q mIL10 mutants are subcloned into pYES2T-ppMF to generate respectively the plasmids pYES2T-mIL10ng1S, pYES2Tng1Q, pYES2T-mIL10ng1S2Q and pYES2T-mIL101Q2Q. These plasmids were introduced in Saccharomyces cerevisiae strain INV S.c.1.

One colony of all the transformants and the vector control *Saccharomyces cerevisiae* INV Sc1[pYES2] were inoculated in 50 ml minimal uracil deficient medium (SD+CSM-U). After 24 hours of aerobic growth at 30°C the cells were pelleted by centrifugation (5 minutes@2500 tmp) and resuspended in YPD medium. At 24 hours, supernatant samples were taken for mIL10 quantification and characterization. During the 48 hours of growth, pH of the *Saccharomyces cerevisiae* supernatant remained stable at pH 7.

Proteins were extracted from the culture supernatant by TCA precipitation and subsequently dissolved in Laemmli sample buffer (Laemmli 1970). Protein fractions were separated by (SDS-PAGE and electroblotted onto a nitrocellulose membrane (Burnette 1981).

Murine interleukin-10 was detected with a polyclonal rabbit anti murine IL-10 as primary antibody at a 1/1000 dilution (Prepro Tech, London, U.K.). The secondary antibody was goat anti rabbit IgG (H+L) coupled to alkaline phosphatase (SBA, Birmingham, USA) and was used at a 1/1000 dilution. Enzymatic activity was revealed with NBT/BCIP substrate (Boehringer Mannheim, GmbH, Germany). Figure 8 shows mIL10 detection in the supernatant of the different *Saccharomyces cerevisiae* INV S.c.1 strains by Western blot after 24 hours of growth in YPD. By changing the 11-NCT-13 Saccharomyces mIL10 glycosylation site to 11-SCT-13 (like in hIL10), we could eliminate the hyperglycosylation of mIL10 by Saccharomyces. Removal of the first potential glycosylation site (11-SCT-13) was sufficient to avoid mIL10 glycosylation. Removal of the second 116-NKS-118 glycosylation site to 116-QKS-118 provided no benefit. In the animal study experiments, the natural mIL10 form and the mIL10ng1S from where the first potential glycosylation site is humanized to 11-SCT-13 were used.

### Example 4: Treatment of chronic DSS colitis with live S. cerevisiae secreting mIL10ng1 S on a plasmid driven way

Therapeutic efficacy of mIL10 secreting *Saccharomyces cerevisiae* strains for the treatment of chronic colitis was evaluated in the dextran sodium sulphate (DSS)-induced mouse model.

Mice were induced to chronic colitis by DSS as described.(Okayasu *et al.,* 1990; Kojouharoff et *al.,* 1997) Daily treatment for 14 days with 2 x 10⁸ CFU *Saccharomyces cerevisiae* INV Sc1 transformants that secrete the non-glycosylated form of mIL10 (*n* = 10; S.c. mIL10ng1S) resulted in a significant lower histological score of the distal colon in comparison with mock (n = 10) and *Saccharomyces cerevisiae* vector control (*n* = 10) treated groups (Figure 9). The efficacy of S.c. mIL10ng1S treatment against established chronic DSS colitis was comparable as that observed with daily treatment for 14 days with 2 x 10⁹ CFU *L. lactis* secreting mIL10 (*n* = 10; LL-mIL10; Figure 9). The non-glycosylated form of mIL10 (mIL10ng1S) that was secreted by *Saccharomyces cerevisiae* performed better than the glycosylated form with regard to the therapeutic efficacy (Figure 9).

### Example 5: Construction of a genetically modified (GM) biologically contained mIL10ng1S secreting Saccharomyces cerevisiae strain

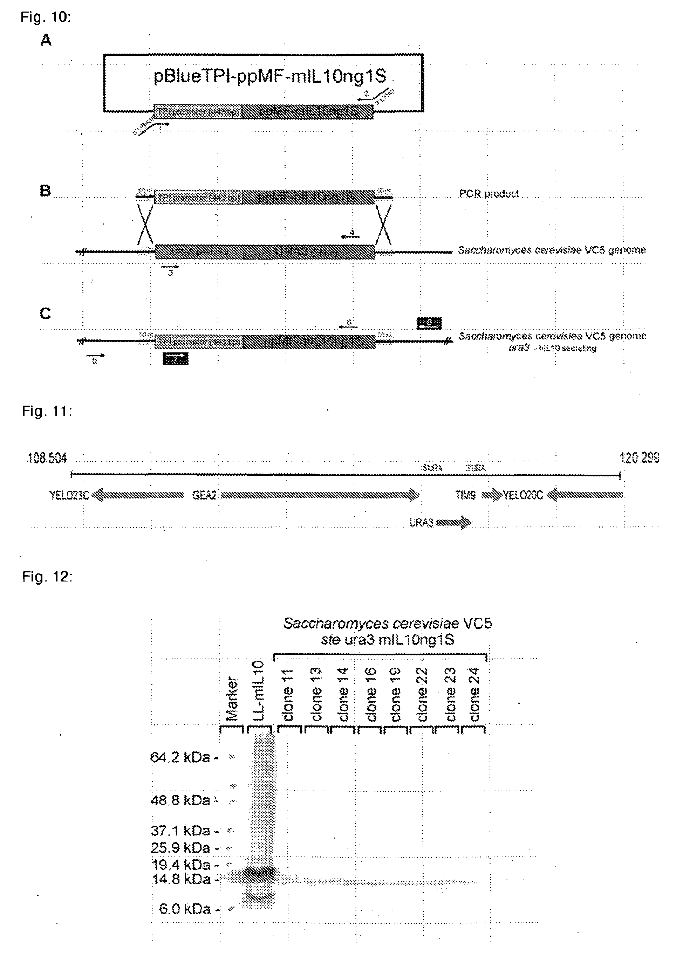

To achieve stable secretion of a therapeutic protein by *Saccharomyces cerevisiae,* it is important that the protein expression cassette is genomically integrated. Under non selective circumstances *Saccharomyces cerevisiae* will rapidly loose its incriminating and non-essential therapeutic gene expressing plasmid and the DNA of the recombinant gene will be spread into nature, which is highly undesirable. It is also important to create a GM *Saccharomyces cerevisiae* strain that is biologically contained and thus cannot survive in the environment. Therefore, a sterile *(ste)* haploid labstrain that is auxotroph (Botstein *et al.,* 1979) was used. Auxotroph yeast strains can only survive in rich medium. In minimal medium or the environment these yeast strains undergo growth arrest after which they die. A haploid, auxotroph yeast strain that is also sterile *(ste)* cannot transfer the DNA of the therapeutic gene to another yeast strain. We used the haploid and sterile *Saccharomyces cerevisiae* Meyen ex E.C. Hansen VC5 strain (MATα, *ste)* (Mackay *et al.,* 1974a; Mackay *et al.,* 1974b) to secrete mIL10. The essential URA3 gene and promotor will be exchanged by homologue recombination by the mIL10ng1S gene, preceded by the strong and constitutive TPI promotor (Figure 10).

The complete orotidine-5'phosphate (OMP) decarboxylase (URA3) gene (Figure 11) including the URA3 promotor was replaced by the constitutive and strong TPI promotor followed by the ppMF-mIL10ng1S DNA fragment. In the ppMF-mIL10ng1S DNA fragment the first glycosylation site is humanized, which circumvents the problem of hyperglycosylation by *Saccharomyces cerevisiae,* and is fused in frame tot the ppMF secreting signal

By replacement of P_{URA3}-URA3 by P_{TPI}-ppMF-mIL10ng1S, we created an *ura3* auxotroph strain. In the absence of uracil this strain undergoes growth arrest and dies. The *Saccharomyces cerevisiae* genome is completely sequenced and made public (Dietrich *et al.,* 1997). Based on the DNA sequence of the URA3 region oligos were developed (oligo 1 = 5'URA3P-TPI-S, and oligo 2 = mIL10-3'URA3-AS,
CTAATTTGTGAGTTTAGTATACATGCATTTACTTATAATACAGTTTTTTAGCTTTTCATTTTG ATCATCATGTATGC on Figure 10A) that allowed amplification of the P_{TPI}-ppMF-hIL10 expression cassette with the addition at the 5' and 3' prime end of the PCR product of respectively 50 nucleotides (nt) of the e 5' en 3' flanking regions of P_{URA3}-URA3 (10B). This PCR fragment was introduced into LiOAc/PEG made competent *Saccharomyces cerevisiae* VC5 cells (Schiestl *et al.,* 1989; Gietz *et al.,* 2001). The 50 nt homologue regions of the PCR fragment with the P_{URA3}-URA3 region allows homologue recombination and replacement of the P_{URA3}-URA3 fragment with the P_{TPI}-ppMF-hIL10 expression cassette. By plating out the yeasts on minimal medium that contains 5-fluoroorotic acid (5-FO), only the yeasts that have replaced P_{URA3}-URA3 by P_{TPI}-ppMF-mIL10ng1S can survive (URA3 transforms 5-FO to toxic 5-fluorouracil). Colonies are further investigated by PCR screening on the presence of P_{TPI}-ppMF-mIL10ng1S (oligo 5-6 and oligo 7-8; Figure 10C) and the absence of P_{URA3}-URA3 (oligo3-4 op 10B) (Oligo 3 = URA3-S, TGCTGCTACTCATCCTAGTC; oligo 4 = URA3-AS, TCATCTCTTCCACCCATGTC; oligo 5 = 5'URA3 flanking-S, ATTGAGGGCGGATTACTACC; oligo 6 = mIL10AS, AGGAGTCGGTTAGCAGTATG; oligo 7 = mIL10-S, GCAGTGGAGCAGGTGAAGAG; oligo 8= 3'URA3 flanking-AS, CGGTTGTTCCGTTTGACTTG).

Absence of growth of the constructed *Saccharomyces cerevisiae* VC5 *ste* ura3- mIL 10⁺ strain in minimal medium without uracil was verified using an automated turbidimeter (Bioscreen). Growth was normal in rich medium.

One colony of the Saccharomyces strains *Saccharomyces cerevisiae* VC5 *ste* ura3⁻ mIL10ng1S⁺ and the vector control *Saccharomyces cerevisiae* VC5 ste ura3- were inoculated in 50 ml YPD. After 24 hours of aerobic growth at 30°C the cells were pelleted by centrifugation (5 minutes@2500 tmp) and resuspended in fresh YPD medium. After another 24 hours, supernatant samples were taken for mIL10 quantification and characterization. During the 48 hours of growth, pH of the *Saccharomyces cerevisiae* supernatant remained stable at pH 7. Proteins were extracted from the culture supernatant by TCA precipitation and subsequently dissolved in Laemmli sample buffer (Laemmli 1970). Protein fractions were separated by SDS-PAGE and electroblotted onto a nitrocellulose membrane (Burnette 1981). Murine interleukin-10 was detected with a polyclonal rabbit anti murine IL-10 as primary antibody at a 1/1000 dilution (Prepro Tech, London, U.K.). A goat anti rabbit IgG (H+L) coupled to alkaline phosphatase (SBA, Birmingham, USA), at a 1/1000 dilution, was used as secondary antibody. Enzymatic activity was revealed with NBT/BCIP substrate (Boehringer Mannheim, GmbH, Germany).

Figure 12 shows mIL10 detection in the *Saccharomyces cerevisiae* VC5 ura3 *ste* mIL10ng1S clones supernatant. *L. lactis* MG1363[pT1mIL10] was used as a positive control. From Figure we can conclude that the constructed GM biologically contained *Saccharomyces cerevisiae* VC5 ura3 *ste* mIL10ng1S clones effectively secrete miL10 into the supernatant and can be used for in vivo IL-10 production and treatment of IBD.

### REFERENCES

- Burnette, W.N. (1981) "Western blotting": electrophoretic transfer of proteins from sodium dodecyl sulphate-polyacrylamide gels to unmodified nitrocellulose and radiographic detection with antibody and radioiodinated protein. Anal Biochem. 122, 195-203.
- Blanquet, S., Antonelli, R., Laforet, L., Denis, S., Marol-Bonnin, S. and Alric, M. (2004). Living recombinant Saccharomyces cerevisiae secreting proteins and peptides as a new drug delivery system in the gut. J. Biotechnol. 110, 37-49.
- Botstein, D, SC Falco, SE Stewart, M Brennan, S Scherer, DT Stinchcomb, K Struhl and RW Davis (1979). "Sterile host yeasts (SHY): a eukaryotic system of biological containment for recombinant DNA experiments." Gene 8(1): 17-24.
- Croxford, J.L., Feldmann, M., Chernajovsky, Y. and Baker, D. (2001). Different therapeutic outcomes in experimental allergic encephalomyelitis dependant upon the mode of delivery of IL-10: a comparison of the effects of protein, adenoviral or retroviraIIL-10 delivery into the central nervous system. J. Immunol. 166, 4124-4130.
- Dietrich, FS, J Mulligan, K Hennessy, MA Yelton, E Allen, R Araujo, E Aviles, A Berno, T Brennan, J Carpenter, E Chen, JM Cherry, E Chung, M Duncan, E Guzman, G Hartzell, S Hunicke-Smith, RW Hyman, A Kayser, C Komp, D Lashkari, H Lew, D Lin, D Mosedale, RW Davis and et al. (1997). "The nucleotide sequence of Saccharomyces cerevisiae chromosome V." Nature 387(6632 Suppl): 78-81.
- Gietz, RD and RA Woods (2001). "Genetic transformation of yeast." Biotechniques 30: 816-20, 822-6, 828 passim.
- Kojouharoff, G., Hans, W., Obermeier, F., Mannel, D.N., Andus, T., Scholmerich, J. Gross, V., and Falk, W. (1997). Neutralization of tumour necrosis factor (TNF) but not of IL-1 reduces inflammation in chronic dextran sulphate sodium-induced colitis in mice. Clin. Exp. Immunol. 107, 353-358.
- Laemmli, U.K. (1970). Cleavage of structural proteins during the assembly of the head of bacteriophage T4. Nature, 227, 680-685.
- Mackay, V and TR Manney (1974a). "Mutations affecting sexual conjugation and related processes in Saccharomyces cerevisiae. I. Isolation and phenotypic characterization of nonmating mutants." Genetics 76: 255-71.
- Mackay, V and TR Manney (1974b). "Mutations affecting sexual conjugation and related processes in Saccharomyces cerevisiae. II. Genetic analysis of nonmating mutants." Genetics 76: 273-88.
- Okayasu, I, S Hatakeyama, M Yamada, T Ohkusa, Y Inagaki and R Nakaya (1990). "A novel method in the induction of reliable experimental acute and chronic ulcerative colitis in mice." Gastroenterology 98: 694-702.
- Sambrook, J., Fritsch, E.F., and Maniatis T. Molecular cloning-a laboratory manual. Cold Spring Harbor Laboratory, New York (1990).
- Santelmann, H. and Howard, J.M. (2005). Yeast metabolic products, yeast antigens and yeasts as possible triggers for irritable bowel syndrome. Eur. J. Gastroenterol. Hepatol. 17,21-26.
- Schiestl, RH and RD Gietz (1989). "High efficiency transformation of intact yeast cells using single stranded nucleic acids as a carrier." Curr Genet 16: 339-46.
- Schotte, L., Steidler, L., Vanderkerckhove, J. and Remaut, E. (2000). Secretion of biologically active murine interleukin-10 by Lactococcus lactis. Enzyme Microb. Technol. 27, 761-765.
- Steidler, L., Hans, W., Schotte, L., Neirynck, S., Obermeirer, F., Falk, W., Fiers, W. and Remaut, E. (2000). Treatment of murine colitis by Lactococcus lactis secreting interleukin-10. Science, 289, 1352-1355
- Vandenbroucke, K., Hans, W., Van Huysse, J., Neirynck, S., Demetter, P., Remaut, E., Rottiers, P., and Steidler, L. (2004). Active delivery of trefoil factors by genetically modified Lactococcus lactis prevents and heals acute colitis in mice. Gastroenterology 127, 502-513.
- Vieira, P, R de Waal-Malefyt, MN Dang, KE Johnson, R Kastelein, DF Fiorentino, JE deVries, MG Roncarolo, TR Mosmann and KW Moore (1991). "Isolation and expression of human cytokine synthesis inhibitory factor cDNA clones: homology to Epstein-Barr virus open reading frame BCRFI." Proc Natl Acad Sci U S A 88: 1172-6.

## Claims

1. Use of a recombinant anti-inflammatory compound producing yeast strain and/or a cytokine antagonist-producing yeast strain for the preparation of a medicament to treat mucosal inflammation, whereby said anti-inflammatory compound or cytokine antagonist is mutated to avoid or limit glycosylation.

2. The use of a recombinant yeast according to claim 1, whereby said mucosal inflammation is an inflammatory bowel disease.

3. The use of a recombinant yeast strain according to claim 1 or 2 wherein the anti-inflammatory compound or cytokine antagonist is selected from the list consisting of IL-10, a trefoil factor such as TFF1, TFF2 or TFF3, a TNF antagonist such as the soluble TNF receptor, INCA, ABIN, an IL-12 antagonist, an Interferon-y antagonist, an IL-1 antagonist and a virus-coded cytokine analogue such as EBV BCRF1.

4. The use of a recombinant yeast according to any of the preceding claims, whereby said anti-inflammatory compound is non-glycosylated IL-10.

5. The use of a recombinant yeast strain according to any of the preceding claims wherein the yeast strain is selected from the group consisting of Saccharomyces sp., Hansenula sp., Kluyveromyces sp. Schizzosaccharomyces sp. Zygosaccharomyces sp., Pichia sp., Monascus sp., Geotrichum sp and Yarrowia sp.

6. Use of a recombinant yeast strain according to claim 5 wherein the Saccaromyces sp is Saccharomyces cerevisiae.

7. Use of a recombinant yeast strain according to claim 6 wherein the recombinant yeast strain is Saccharomyces cerevisiae subspecies boulardii.

8. Use of a recombinant yeast strain according to any of the preceding claims wherein the inflammatory bowel disease is a chronic colitis, Crohn's disease or an ulcerative colitis.

## Patentansprüche

1. Verwendung eines eine rekombinante entzündungshemmende Verbindung produzierenden Hefestamms und/oder eines einen Cytokin-Antagonisten produzierenden Hefestamms für die Herstellung eines Arzneimittels zur Behandlung von Schleimhautentzündung, wobei die entzündungshemmende Verbindung bzw. der Cytokin-Antagonist zur Vermeidung oder Beschränkung von Glykosylierung mutiert ist.

2. Verwendung einer rekombinanten Hefe nach Anspruch 1, wobei es sich bei der Schleimhautentzündung um eine entzündliche Darmkrankheit handelt.

3. Verwendung eines rekombinanten Hefestamms nach Anspruch 1 oder 2, wobei die entzündungshemmende Verbindung bzw. der Cytokin-Antagonist aus der aus IL-10, einem Kleeblatt-Faktor, wie etwa TFF1, TFF2 oder TFF3, einem TNF-Antagonisten, wie etwa dem löslichen TNF-Rezeptor, INCA, ABIN, einem IL-12-Antagonisten, einem Interferon-γ-Antagonisten, einem IL-1-Antagonisten und einem viruscodierten Cytokinanalog, wie etwa EBV-BCRF1 bestehenden Liste ausgewählt ist.

4. Verwendung einer rekombinanten Hefe nach einem der vorhergehenden Ansprüche, wobei es sich bei der entzündungshemmenden Verbindung um nicht glykosyliertes IL-10 handelt.

5. Verwendung eines rekombinanten Hefestamms nach einem der vorhergehenden Ansprüche, wobei der Hefestamm aus der aus Saccharomyces sp., Hansenula sp., Kluyveromyces sp., Schizosaccharomyces sp., Zygosaccharomyces sp., Pichia sp., Monascus sp., Geotrichum sp. und Yarrowia sp. bestehenden Gruppe ausgewählt ist.

6. Verwendung eines rekombinanten Hefestamms nach Anspruch 5, wobei es sich bei Saccharomyces sp. um Saccharomyces cerevisiae handelt.

7. Verwendung eines rekombinanten Hefestamms nach Anspruch 6, bei dem es sich um Saccharomyces cerevisiae subspecies boulardii handelt.

8. Verwendung eines rekombinanten Hefestamms nach einem der vorhergehenden Ansprüche, wobei es sich bei der entzündlichen Darmkrankheit um eine chronische Colitis, Morbus Crohn oder Colitis ulcerosa handelt.

## Revendications

1. Utilisation d'une souche de levure recombinante productrice de composé anti-inflammatoire et/ou d'une souche de levure productrice d'antagoniste de cytokines pour la préparation d'un médicament destiné à traiter l'inflammation des muqueuses, ledit composé anti-inflammatoire ou antagoniste de cytokines étant muté pour éviter ou limiter la glycosylation.

2. Utilisation d'une levure recombinante selon la revendication 1, ladite inflammation des muqueuses étant une maladie inflammatoire de l'intestin.

3. Utilisation d'une souche de levure recombinante selon la revendication 1 ou 2, dans laquelle le composé anti-inflammatoire ou l'antagoniste de cytokines est choisi dans la liste constituée de IL-10, d'un facteur en feuille de trèfle tel que TFF1, TFF2 ou TFF3, d'un antagoniste de TNF tel que le récepteur soluble du TNF, de INCA, de ABIN, d'un antagoniste de IL-12, d'un antagoniste d'interféron-γ, d'un antagoniste de IL-1 et d'un analogue de cytokine codé par un virus tel que BCRF1 de EBV.

4. Utilisation d'une levure recombinante selon l'une quelconque des revendications précédentes, ledit composé anti-inflammatoire étant IL-10 non glycosylé.

5. Utilisation d'une souche de levure recombinante selon l'une quelconque des revendications précédentes, dans laquelle la souche de levure est choisie dans le groupe constitué de Saccharomyces sp. , de Hansenula sp. , de Kluyveromyces sp. , de Schizzosaccharomyces sp. , de Zygosaccharomyces sp. , de Pichia sp. , de Monascus sp. , de Geotrichum sp. et de Yarrowia sp.

6. Utilisation d'une souche de levure recombinante selon la revendication 5, dans laquelle Saccharomyces sp. est Saccharomyces cerevisiae.

7. Utilisation d'une souche de levure recombinante selon la revendication 6, dans laquelle la souche de levure recombinante est Saccharomyces cerevisiae sous-espèce boulardii.

8. Utilisation d'une souche de levure recombinante selon l'une quelconque des revendications précédentes, dans laquelle la maladie inflammatoire de l'intestin est une colite chronique, la maladie de Crohn ou une colite ulcéreuse.
